# EUROPEAN PATENT APPLICATION

(11) **EP 4 534 079 A1**
(43) Date of publication of application: **09.04.2025**
(21) Application number: 24204139.0
(22) Date of filing: 02.10.2024
(51) Int. Cl.: A61K 31/047, A61K 9/00, A61K 31/353, A61K 33/00, A61K 31/6615, A61P 3/00, A61P 9/00

(54) **COMPOSITION BASED ON INOSITOL AND OLIGOMERIC PROANTHOCYANIDINS**

(30) Priority: 06.10.2023 IT 202300020718
(71) Applicant: Promin Prodotti di Medicina Integrata S.r.l., 20032 Cormano (MI) (IT)
(72) Inventor: Pialorsi, Federico, 20032 Cormano (MI) (IT)
(74) Representative: Tagliafico, Giulia

(57) **Abstract**

A composition based on inositol, oligomeric proanthocyanidins and chromium for the prevention and/or treatment of metabolic syndrome and for the primary prevention of cardiovascular diseases, particularly in premenopausal women, is described here.

## Description

### FIELD OF THE INVENTION

The present invention relates to a composition based on inositol and oligomeric proanthocyanidins for the prevention and treatment of metabolic syndrome and for the primary prevention of cardiovascular diseases, particularly in premenopausal women.

### BACKGROUND OF THE INVENTION

The term "metabolic syndrome" does not refer to a single pathology, but to a set of risk factors linked to conditions that increase the possibility of developing cerebrovascular and cardiovascular diseases and diabetes.

In fact, patients diagnosed with metabolic syndrome have a two-times greater risk of developing heart disease and a five-times greater risk of developing diabetes. It is a clinical condition that affects about half of the population over the age of 50-60 and its incidence is constantly growing.

The conditions that predispose to the development of metabolic syndrome are different and are the following: presence of an excessive amount of body fat, especially at the abdominal level, the so-called visceral fat with variation in the weight-height ratio (so-called Body mass index BMI), but also linked to excessive waist circumference; high levels of LDL cholesterol and triglycerides in the blood; arterial hypertension (blood pressure values>140/90); low levels of HDL cholesterol (the so-called "good" cholesterol); insulin resistance, a hormone that helps regulate the amount of sugar present in the body at the peripheral level in the so-called target organs (liver, muscle, adipose tissue) with consequent hyperglycemia; and hyperuricemia.

The higher the number of conditions a patient suffers from, the greater the likelihood of developing metabolic syndrome. It should also be noted that all these conditions are related: excess abdominal fat, in fact, can cause an alteration in the metabolism of sugars and fats and the activation of a chronic inflammation process that contributes to the possible development of atherosclerosis in the blood vessels, with consequent risk of cardiovascular diseases.

Furthermore, the risk of developing metabolic syndrome increases with age; if you suffer from diabetes, you are more predisposed to developing this syndrome; genetic predisposition and lack of physical exercise can play a role of some importance in the onset of the syndrome. Myo-inositol is a cyclitol naturally present in animal and plant cells, both in its free form and as a bound component of phospholipids or inositol phosphate derivatives. It plays an important role in various cellular processes and for this reason myo-inositol is essential or important for the proper working of a wide range of cellular functions, including cell growth and survival, peripheral nerve development and function, osteogenesis and reproduction. Several isomers of inositol, particularly myo-inositol (MI) and D-chiro-inositol (DCI), have been shown to possess insulin-mimetic properties and to be effective in lowering postprandial glycemia. Furthermore, abnormalities in inositol metabolism are associated with insulin resistance and long-term microvascular complications of diabetes, supporting a role for inositol or its derivatives in glucose metabolism.

Moreover, when administered as dietary supplements, both myo- and d-chiro-inositol have shown insulin-mimetic effects in several animal models of insulin resistance and in women with polycystic ovary syndrome, a metabolic and endocrine disorder associated with insulin resistance.

The potential benefits of a dietary supplement containing myo-inositol, by far the most common inositol isomer in food products, in human disorders associated with insulin resistance (polycystic ovary syndrome, gestational diabetes mellitus or metabolic syndrome) or in the prevention or treatment of some diabetic complications (neuropathy, nephropathy, cataracts) have already been reported (Marine L. Croze et al., Biochimie, 95 (10), 1811-1827, 2013).

Myo-inositol supplementation, administered from early pregnancy, has also been shown to reduce the incidence of gestational mellitus diabetes in overweight non-obese women (Angelo Santamaria et al., The Journal of Maternal-Fetal & Neonatal Medicine, 29:19, 3234-3237, 2016).

Inositol has been reported to improve insulin sensitivity because it functions as a second messenger of FSH, achieving insulin-like effects on metabolic enzymes. A study evaluated the efficacy of myo-inositol on FSH levels in postmenopausal women. Myo-inositol administration induced a decrease in FSH levels after three months of treatment, as well as during the second trimester of supplementation compared to baseline (Giuseppe Gullo et al., Minerva Ginecologica 67(5):485-486, 2015). The authors of the study concluded from these results that myo-inositol could be considered a valid insulin-sensitizing molecule for the management of menopausal disorders.

Oligomeric proanthocyanidins [OPC], also known as proanthocyanidin oligomers, proanthocyanidins, and procyanidins) are a class of polyphenols present in grape seeds. OPCs are formed by catechins and epicatechins linked together to form a typical oligomer. They are found in some plants - such as maritime pine, horse chestnut, blueberry and cypress - and in particularly high concentrations in *Vitis vinifera L.* grapes. OPCs have been brought up about the interpretation of the so-called "French paradox", according to which the French - despite habitually eating foods considered atherogenic - are relatively more protected than expected given their eating habits, since they do not easily give up wine. This "paradox" - although, after thirty years, it is currently under review - has inspired several studies, which together have helped to identify the indisputable protective effect of OPCs against age-related, cardiovascular and neurodegenerative diseases, as well as diabetes, whose common thread is a modulation of the inflammatory process associated with a particular tropism for the vascular system.

OPCs are immediately bioavailable, already 10 minutes after administration they are present in the blood, with a peak at 45 minutes. OPCs are able to protect tissues from the degradation of collagen and elastin (important proteins of connective tissue) and to promote the formation of collagen microfibrils.

In addition, OPCs protect against damage from free radicals; they have antioxidant, immunostimulant and antimutagenic action; they are useful in cases of edema and inflammation. Their use is mainly aimed at protecting blood vessels and strengthening microcirculation, for this reason OPC extracts are administered to people suffering from chronic venous insufficiency, varicose veins, hemorrhoidal syndrome and problems related to circulatory disorders in general. The recommended doses in chronic and significant cases are high, reaching up to 95% titration in OPC in 100-300 mg of dry grape seed extract.

A new pharmaceutical standard supplement of grape seed extract (ECOVITIS^{®}) has been shown to prevent and control borderline hypertension and endothelial dysfunction.

### DESCRIPTION OF THE INVENTION

The Applicant has now found that a combination of inositol and oligomeric proanthocyanidins shows a synergistic effect in the prevention and treatment of metabolic syndrome and in the primary prevention of cardiovascular diseases, particularly in premenopausal women.

One aspect of the invention relates to a composition for pharmaceutical or nutritional use comprising inositol and oligomeric proanthocyanidins, together with one or more pharmaceutically acceptable excipients, useful in the prevention and treatment of metabolic syndrome and in the primary prevention of cardiovascular diseases, particularly in premenopausal women.

According to one aspect of the invention, inositol is selected from the group comprising myo-inositol, D-chiro-inositol and inositol triphosphate.

According to another aspect of the invention, oligomeric proanthocyanidins are contained in grape seed extracts. There are several commercial plant extracts of grape seeds, which contain high percentages of oligomeric proanthocyanidins, preferably greater than 80% by weight, more preferably greater than 90% and even more preferably greater than 95%. Preferably, the oligomeric proanthocyanidins of the present invention are contained in the grape seed extract, marketed under the trademark ECOVITIS^{®}. This extract has the advantage of providing the oligomeric proanthocyanidins in a formulation that is more easily absorbed at the intestinal level.

According to another aspect of the invention, the composition of the invention also comprises chromium.

Another object of the present invention is, therefore, a method for the prevention and treatment of metabolic syndrome and for the primary prevention of cardiovascular diseases, in particular in premenopausal women, by administering a composition for pharmaceutical or nutritional use, which comprises inositol and oligomeric proanthocyanidins, together with one or more pharmaceutically acceptable excipients.

The term "effective amount" means an amount of active ingredients that is sufficient to influence the prevention and treatment of metabolic syndrome and for the primary prevention of cardiovascular diseases. The effective amount will depend on the route of administration, the dose and the condition of the patient.

According to the present invention, the term "treatment" means activities intended to cure, alleviate the symptoms or delay the progression of a disease or pathological condition.

The term "prevention" refers to activities aimed at minimizing the incidence or effects of a disease or pathological condition or preventing the onset of a disease in subjects at risk, such as the reduction of risk factors, but also to halt the evolution of a disease that has already occurred and reduce its consequences.

In particular, the term "primary prevention of cardiovascular disease" or "primary cardiovascular prevention" refers to activities aimed at minimizing the risk that a person who has not previously had a disease of the heart or blood vessels may begin to suffer from it.

In general, pharmaceutical compositions, as contemplated in accordance with the present invention, comprise effective amounts of the above-mentioned extracts together with pharmaceutically acceptable diluents, stabilizers, preservatives, solubilizers, emulsifiers, adjuvants and/or carriers; see, e. g., Remington of Pharmaceutical Sciences, 18th Ed. (1990, Mack Publishing Co., Easton, Pa. 18042) pages 1435-712.

An effective amount of active ingredient can be readily determined by a person skilled in the art taking into consideration factors such as body weight, age, desired therapeutic or prophylactic target.

According to another aspect of the invention an effective daily amount of inositol for oral administration is between 500 mg and 5,000 mg. According to a preferred aspect the effective amount is between 1,000 and 3,000 mg, more preferably 2,000 mg.

According to one aspect of the invention, an effective daily amount of oligomeric proanthocyanidins for oral administration is between 30 mg and 1,000 mg. According to a preferred aspect, the effective amount is between 100 mg and 600 mg, more preferably 300 mg.

The grape seed extracts present on the market report a content of oligomeric proanthocyanidins, which varies from 10% to 98%, in particular 50% to 85%.

According to one aspect of the invention, an effective daily amount of chromium for oral administration is between 10 µg and 300 µg, preferably between 20 µg and 100 µg, more preferably 40 µg. According to one aspect of the invention, an effective amount of chromium is 100% of the NRV (Nutritional Reference Value). The 100% NRV value corresponds to the daily amount of a substance, which can be provided with a food supplement.

The effective daily amounts for oral administration reported above can be divided into 2 or 4 administration units (e.g. tablets) per day.

One aspect of the invention is, therefore, a composition comprising inositol and oligomeric proanthocyanidins, together with one or more pharmaceutically acceptable excipients, for use in the prevention and/or treatment of metabolic syndrome and in the primary prevention of cardiovascular disease, particularly in premenopausal women, wherein the inositol is present in an amount of between 100 mg and 5,000 mg, optionally between 200 and 2,000 mg, more optionally 500 mg.

A further aspect of the invention is, therefore, a composition comprising inositol and oligomeric proanthocyanidins, together with one or more pharmaceutically acceptable excipients, for use in the prevention and/or treatment of metabolic syndrome and in the primary prevention of cardiovascular diseases, particularly in premenopausal women, wherein the oligomeric proanthocyanidins are present in an amount between 30 mg and 1,000 mg, optionally between 50 mg and 300 mg, more preferably 75 mg.

Another aspect of the invention is, therefore, a composition comprising inositol and oligomeric proanthocyanidins, together with one or more pharmaceutically acceptable excipients, for use in the prevention and/or treatment of metabolic syndrome and in the primary prevention of cardiovascular disease, particularly in premenopausal women, wherein chromium is present in an amount between 5 µg and 300 µg, optionally between 7 µg and 50 µg, plus optionally 40 µg.

Another aspect of the invention, is a composition comprising inositol, oligomeric proanthocyanidins and chromium, together with one or more pharmaceutically acceptable excipients, for use in weight loss and for the prevention and/or treatment of weight gain.

The term "pharmaceutically acceptable" is intended to include any excipient, which does not interfere with the efficacy of the biological activity of the active ingredient and which is not toxic to the "host" to which it is administered.

The administration of the composition of the present invention is preferably by oral route. Other routes of administration, which establish the desired blood levels of the respective ingredients, are included in the present invention.

The term "excipient" herein refers to conventional excipients, i.e. compounds that are inert with respect to the active ingredient and the pharmaceutical form. Examples of different classes of such excipients are: diluents (compounds added when the mass of the active ingredient is not sufficient for the preparation of the composition); lubricants (which prevent powders from adhering to mechanical parts during the production process); aggregating agents (compounds that increase the cohesion of powders); colorants (used to improve the presentation of some pharmaceutical forms, for example capsules, or to classify them according to the therapeutic category to which they belong or to distinguish them from other similar products); sweeteners or flavorings (added to improve the organoleptic characteristics of the products); antioxidants-antimicrobials (used to extend the shelf life of the product.

The most commonly used conventional excipients are: glyceryl behenate, magnesium stearate, magnesium oxide, hydrated silica, amorphous silica, maltodextrin, microcrystalline cellulose, maltitol, mannitol, xylitol, flavourings, sucralose, rice starch and maltose.

In addition to the pharmaceutically acceptable excipients, the composition of the invention may also comprise smaller amounts of additives, such as stabilizers, buffers and preservatives.

The composition of the present invention is preferably formulated in the form of tablets or capsules or sachets.

According to one aspect of the invention, the composition of the present invention is formulated in tablets, preferably slow-release tablets. To obtain slow-release tablets, either coating systems with methacrylates (pH-sensitive lacquer) or other paints available on the market can be used, or through co-milling matrix systems with appropriate excipients, such as cross-linked (or cross-linked) cellulose or lipophilic excipients, such as fatty acids (for example stearic acid and palmitic acid).

A further object of the present invention is a food supplement containing the composition of the present invention in one of the pharmaceutical forms previously illustrated.

The term "food supplement" means a food product intended to supplement the common diet and which constitutes a concentrated source of nutrients, such as vitamins and minerals, or other substances having a nutritional or physiological effect, in pre-dosed forms (see also Directive 2002/46/EC of 10 June 2002 and Legislative Decree 21 May 2004 no. 169 art. 2).

A further object of the present invention is a food for special medical purposes containing the composition of the present invention in one of the pharmaceutical forms previously illustrated.

The term "food for special medical purposes" (pursuant to Regulation (EU) 609/2013) refers to products intended for the dietary treatment of subjects affected by disorders, diseases or medical conditions that determine nutritional vulnerability, i.e. the impossibility or severe difficulty in feeding themselves using common foods, including food supplements, to satisfy their nutritional needs.

The production process of the composition of the present invention takes place using well-known technologies and operating conditions. In general, the process comprises mixing the active ingredients (or plant extracts containing them) together with the excipients and other necessary components in a mixer in order to obtain the desired composition.

For example, the process schematically comprises the following steps: 1) weighing the active components and excipients necessary to obtain the desired release, 2) mixing all the components in a mixer (for example a rotating screw), 3) compressing the mixture obtained and subsequent painting with transparent, colored and/or gas-resistant paint or using the mixture obtained in step 2 directly packaged in sachets or capsules. In the case of poorly flowing powders, a pre-compression and subsequent dry granulation through an oscillating granulator can be carried out before compressing. Some examples (not limiting) of the present invention are provided below.

### EXAMPLES

### EXAMPLES 1-10

The following Table 1 reports some Examples of food supplement according to the present invention. For each Example, the active ingredients and/or plant extracts used, the respective amounts per unit dose (in milligrams) and the pharmaceutical form used are reported.

The amounts of components (intended per unit dose of tablet) reported in Table 1 were weighed separately and subsequently mixed in a rotating screw mixer.

**Table 1**

| **EXAMPLE 1** | | **PHARMACEUTICAL FORM** |
|---|---|---|
| ACTIVE INGREDIENT | mg/CPR | SLOW RELEASE TABLET |
| Ecovitis^{®} | 75 | |
| Myo-inositol | 500 | |
| Chromium | 10 (mcg) | |
| | | |

| **EXAMPLE 2** | | **PHARMACEUTICAL FORM** |
|---|---|---|
| ACTIVE INGREDIENT | mg/CPR | SLOW RELEASE TABLET |
| Ecovitis^{®} | 150 | |
| Myo-inositol | 1.000 | |
| Chromium | 40 (mcg) | |
| | | |

| **EXAMPLE 3** | | **PHARMACEUTICAL FORM** |
|---|---|---|
| ACTIVE INGREDIENT | mg/CPR | OROSOLUBLE TABLET |
| Ecovitis^{®} | 300 | |
| Myo-inositol | 2.000 | |
| Chromium | 100 (mcg) | |
| | | |

| **EXAMPLE 4** | | **PHARMACEUTICAL FORM** |
|---|---|---|
| ACTIVE INGREDIENT | mg/CPR | GRANULES PER SACHET |
| Ecovitis^{®} | 600 | |
| Myo-inositol | 4.000 | |
| Chromium | 300 (mcg) | |
| | | |

| **EXAMPLE 5** | | **PHARMACEUTICAL FORM** |
|---|---|---|
| ACTIVE INGREDIENT | mg/CPR | SWALLOWABLE CAPSULE |
| Ecovitis^{®} | 75 | |
| Myo-inositol | 250 | |
| **Chromium** | **20 (mcg)** | |
| | | |

| **EXAMPLE 6** | | **PHARMACEUTICAL FORM** |
|---|---|---|
| ACTIVE INGREDIENT | mg/CPR | SWALLOWABLE CAPSULE |
| Ecovitis^{®} | 75 | |
| Myo-inositol | 250 | |
| Monacolin K | 1,2 | |
| Chromium | 20 (mcg) | |
| | | |

| **EXAMPLE 7** | | **PHARMACEUTICAL FORM** |
|---|---|---|
| ACTIVE INGREDIENT | mg/CPR | SWALLOWABLE TABLET |
| Ecovitis^{®} | 150 | |
| Myo-inositol | 500 | |
| Hawthorn | 250 | |
| Chromium | 20 (mcg) | |
| | | |

| **EXAMPLE 8** | | **PHARMACEUTICAL FORM** |
|---|---|---|
| ACTIVE INGREDIENT | mg/CPR | SWALLOWABLE TABLET |
| Ecovitis^{®} | 150 | |
| Myo-inositol | 500 | |
| Bergamot | 500 | |
| Chromium | 20 (mcg) | |
| | | |

| **EXAMPLE 9** | | **PHARMACEUTICAL FORM** |
|---|---|---|
| ACTIVE INGREDIENT | mg/CPR | SWALLOWABLE TABLET |
| Ecovitis^{®} | 150 | |
| Myo-inositol | 500 | |
| Coenzyme Q10 | 50 | |
| Chromium | 10 (mcg) | |
| | | |

| **EXAMPLE 10** | | **PHARMACEUTICAL FORM** |
|---|---|---|
| ACTIVE INGREDIENT | mg/CPR | GRANULES PER SACHET |
| Ecovitis^{®} | 300 | |
| Myo-inositol | 2.000 | |
| Lipoic Acid | 300 | |
| Chromium | 100(mcg) | |

### EXAMPLE 11- Observational study

To prove the biological action of the composition of the invention with respect to the prevention and treatment of metabolic syndrome and the primary prevention of cardiovascular diseases, the following observational study was carried out on 15 healthy patients, 5 men and 10 women, all with borderline parameters with respect to the 4 risk factors that characterize metabolic syndrome:
- Fat mass
- Pressure
- Cholesterol
- Glycemia

The patients were visited at T0 and all cardiometabolic parameters were evaluated.

The patients were given an isocaloric diet and correct nutritional information to follow for a period of 3 months.

The patients were divided into two groups:
- Group 1: only advice on an isocaloric diet and nutritional information
- Group 2: Isocaloric diet + nutritional information + regular intake of:
   4 tablets according to Example 1 of the invention for 15 days
   2 tablets according to Example 1 of the invention for 45 days.

After 3 months, the parameters of both groups were measured.

The following results were found, which were considered significant and statistically convincing.
- Reduction of fat mass: Group 2 > 10% compared to Group 1
- Reduction of blood pressure: Group 1: 5% reduction; Group 2 25% reduction
- Reduction of total cholesterol: Group 1: 3% reduction; Group 2 10 to 20% reduction
- Reduction of blood sugar: Group 1: not statistically significant change; Group 2 reduction between 10 and 15%.

## Claims

1. A composition comprising inositol, oligomeric proanthocyanidins and chromium, together with one or more pharmaceutically acceptable excipients, for use in the prevention and/or treatment of the metabolic syndrome and the primary prevention of cardiovascular diseases, particularly in premenopausal women.

2. The composition according to claim 1, wherein inositol is selected from the group comprising myo-inositol, D-chiro-inositol and inositol triphosphate.

3. The composition according to anyone of the preceding claims, wherein inositol is present at an amount comprised between 100 mg and 5,000 mg, optionally between 200 and 2,000 mg, optionally of 500 mg.

4. The composition according to anyone of the preceding claims, wherein the oligomeric proanthocyanidins are contained in a grape seed extract.

5. The composition according to claim 4, wherein the grape seed extract comprises an amount of oligomeric proanthocyanidins comprised between 10% and 98% by weight of the extract.

6. The composition according to anyone of the claims 4 or 5, wherein the grape seed extract comprises an amount of oligomeric proanthocyanidins comprised between 50% and 85% by weight of the extract.

7. The composition according to anyone of the claims from 4 to 6, wherein the oligomeric proanthocyanidins are present at an amount comprised between 30 mg and 1,000 mg, optionally between 50 mg and 300 mg, more optionally of 75 mg.

8. The composition according to anyone of the preceding claims, wherein chromium is present at an amount comprised between 5 µg e 300 µg, optionally between 7 µg e 50 µg, more optionally of 40 µg.

9. A dietary supplement comprising the composition according to anyone of the claims from 1 to 8, for use in the prevention and/or treatment of the metabolic syndrome and the primary prevention of cardiovascular diseases, particularly in premenopausal women.

10. A food for special medical purposes comprising the composition according to anyone of the claims from 1 to 8, for use in the prevention and/or treatment of the metabolic syndrome and primary prevention of cardiovascular diseases, particularly in premenopausal women.
